# EUROPÄISCHE PATENTANMELDUNG

(11) **EP 1 055 656 A2**
(43) Veröffentlichungstag der Anmeldung: **29.11.2000**
(21) Anmeldenummer: 00110973.5
(22) Anmeldetag: 26.05.2000
(51) Int. Cl.: C07C 29/42, C07C 33/042, C07C 33/14, C07C 33/44, C07C 41/30, C07C 43/196, C07C 209/68, C07C 215/24, C07C 215/26

(54) **Verfahren zur Herstellung von Alkin-Verbindungen**

(30) Priorität: 26.05.1999 DE 19924049
(71) Anmelder: BASF AKTIENGESELLSCHAFT, 67056 Ludwigshafen (DE)
(72) Erfinder: Koradin, Christopher, 35260 Stadtallendorf (DE); Aisca Bayeto, Juan Jose, Dr., 68167 Mannheim (DE); Henkelmann, Jochem, Dr., 68165 Mannheim (DE); Preiss, Thomas, Dr., 67256 Weisenheim am Sand (DE); Knochel, Paul, Prof., Dr., 81475 München (DE); Tzelis, Dimitrios, Dr., 35043 Marburg (DE)
(74) Vertreter: Isenbruck, Günter, Dr.

(57) **Zusammenfassung**

CsOH · H₂O wird als Katalysator bei der Alkinylierung von organischen Verbindungen verwendet, die Gruppen >C=O enthalten, durch Umsetzung der Gruppen mit organischen Verbindungen, die die Gruppe -C≡C-H enthalten.

## Beschreibung

Die Erfindung betrifft Verfahren zur Herstellung von Alkin-Verbindungen durch Umsetzung von Verbindungen, die Carbonyl-Gruppen aufweisen, mit Verbindungen, die eine Acetylengruppe (-C≡C-H) enthalten, in Gegenwart von basischen Katalysatoren.

Die Basen-katalysierte Bildung von neuen Kohlenstoff-Kohlenstoff-Bindungen ist ein wichtiges Synthesewerkzeug. Besonders attraktiv in dieser Kategorie von Umsetzungen sind Additionen an Kohlenstoff-Kohlenstoff-Mehrfachbindungen, die ohne die Bildung von Seitenprodukten durchgeführt werden können und damit atomökonomisch sind. Beispielsweise ist die metall-katalysierte Addition von Alkinen an Carbonyl-Verbindungen zur Herstellung von Propargylalkoholen von technischer Bedeutung. Die Durchführung dieser Umsetzungen unter Verwendung einer stöchiometrischen Menge an Base wie Organolithium- oder Organomagnesium-Verbindungen zur Bildung eines Metallacetylid-Zwischenproduktes ist seit langem bekannt. Wesentlich weniger beschrieben ist die katalytische Aktivierung eines Alkins und nachfolgende Addition an Carbonyl-Verbindungen.

In W. Reppe, Liebigs Ann. Chem. 1955, 596, Seiten 1 bis 3, wird die Carbonylierung von Acetylen mit Carbonyl-Verbindungen in Gegenwart von Acetyliden der Schwermetalle der ersten Nebengruppe des Periodensystems der Elemente in THF beschrieben.

Auf den Seiten 6 bis 11 und 25 bis 38 wird beschrieben, daß die Ethinylierung von Ketonen in wäßrigen Medien unter Verwendung von Alkalihydroxiden, Erdalkalihydroxiden, Alkalicarbonaten oder tertiären Aminen als Katalysatoren durchgeführt werden kann. Zudem kann Kupferacetylid als Katalysator verwendet werden. In den Beispielen wird die Herstellung von Methylbutinol aus Aceton und Acetylen in Wasser mit Kaliumhydroxid als Katalysator erwähnt.

R. J. Tedeschi et al., J. Org. Chem. 1963, 28, Seiten 1740 bis 1743 beschreiben die Basen-katalysierte Umsetzung von Acetylen und Phenylacetylen mit Carbonyl-Verbindungen in flüssigem Ammoniak unter Druck. Dabei wird die Umsetzung zu den entsprechenden sekundären und tertiären acetylenischen Carbinolen unter Verwendung katalytischer Mengen von Natrium- oder Kaliumhydroxid durchgeführt.

Derartige Umsetzungen sind in den meisten Fällen auf Acetylen selbst beschränkt oder nur auf bestimmte Carbonyl-Verbindungen anwendbar.

J. H. Babler et al., J. Org. Chem. 1996, 61, Seiten 416 bis 417 beschreiben die Alkoxid-katalysierte Addition von terminalen Alkinen an Ketone. Dabei wird die Umsetzung mit tert.-Butoxid als Katalysator in DMSO als Lösungsmittel durchgeführt.

J. Busch-Petersen et al., Tetrahedron Lett. 1999, Seiten 2065 bis 2068 beschreiben die Verwendung von Cäsiumfluorid als Desilylierungsmittel bei der Herstellung von Carbinolen aus Ketonen und silylierten Acetylenverbindungen.

Dimethylsulfoxid (DMSO) ist für technische Verfahren unvorteilhaft, da es kostspielig und toxikologisch nicht unbedenklich sowie thermisch instabil ist.

Aufgabe der vorliegenden Erfindung ist die Bereitstellung eines verbesserten und allgemein verwendbaren Verfahrens zur Herstellung von Alkin-Verbindungen durch Umsetzung von organischen Verbindungen, die Carbonyl-Gruppen aufweisen, mit Verbindungen, die acetylenische Gruppen (-C≡C-H) aufweisen.

Die Aufgabe wird erfindungsgemäß gelöst durch ein Verfahren zur Herstellung von Alkin-Verbindungen der allgemeinen Formel (I)

R¹R²C(OH)-C≡C-R³ (I)

mit der Bedeutung
- R¹, R², R³: unabhängig H oder C₁₋₂₀-Kohlenwasserstoffrest, der ein- oder mehrfach durch C₁₋₆-Alkyl und/oder Halogen substituiert und/oder von nicht benachbarten Heteroatomen unterbrochen sein und/oder C-C-Doppelbindungen enthalten kann, wobei mindestens einer der Reste R¹ und R² von H verschieden ist,
durch Umsetzung von Verbindungen der allgemeinen Formel (II)

R¹-C(=O)-R² (II)

mit Verbindungen der allgemeinen Formel (III)

H-C≡C-R³ (III)

in Gegenwart eines basischen Katalysators, wobei als basischer Katalysator CsOH · H₂O eingesetzt wird und/oder die Umsetzung in einem Lösungsmittel auf Basis von N-Methylpyrrolidon (NMP) durchgeführt wird.

Es wurde erfindungsgemäß gefunden, daß die Umsetzung für eine Vielzahl von Verbindungen mit verbesserter Katalysatoraktivität und Ausbeute durchgeführt werden kann, wenn als basischer Katalysator CsOH · H₂O eingesetzt wird. Die Umsetzung kann dabei lösungsmittelfrei oder in geeigneten Lösungsmitteln erfolgen. Besonders bevorzugt ist die Umsetzung in einem Lösungsmittel auf Basis von N-Methylpyrrolidon (NMP). Der Ausdruck 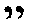 auf Basis von" bedeutet, daß auch Lösungsmittelgemische eingesetzt werden können, die im wesentlichen NMP enthalten. Beispielsweise können Mengen von bis zu 20 Gew.-%, vorzugsweise bis zu 10 Gew.-%, insbesondere bis zu 5 Gew.-% anderer Lösungsmittel toleriert werden. Damit weist das Lösungsmittel vorzugsweise einen Gehalt von mindestens 80 Gew.-%, besonders bevorzugt mindestens 90 Gew.-%, insbesondere mindestens 95 Gew.-% NMP auf. Der verbleibende Rest des Lösungsmittels ist dabei gegenüber den Umsetzungen inert. Beispiele sind DMSO und THF sowie Gemische davon. Speziell bevorzugt wird im wesentlichen oder ganz ausschließlich NMP als Lösungsmittel eingesetzt.

Die Verwendung von CsOH · H₂O ist auch im Zusammenhang mit anderen Lösungsmitteln wie DMSO, THF oder Gemischen aus THF und DMSO oder unter Verzicht auf Lösungsmittel möglich. Besonders gute Ergebnisse erzielt man jedoch bei einer Kombination von CsOH · H₂O mit NMP als Lösungsmittel.

Entsprechend ist die Umsetzung in einem Lösungsmittel auf der Basis von NMP mit einer Vielzahl basischer Katalysatoren möglich. Vorzugsweise wird die Umsetzung in Gegenwart von Alkalihydroxiden, besonders bevorzugt in Gegenwart von Natriumhydroxid, Kaliumhydroxid oder Cäsiumhydroxid, insbesondere in Gegenwart von Cäsiumhydroxid durchgeführt.

Als Alkin werden Verbindungen der allgemeinen Formel (III)

H-C≡C-R³ (III)

eingesetzt mit der vorstehenden Bedeutung für R³.

Insbesondere können neben Acetylen und Phenylacetylen auch fünktionalisierte Alkine wie Propargylamin eingesetzt werden.

Das erfindungsgemäße Katalysatorsystem wird, bezogen auf die Alkaliverbindung, vorzugsweise CsOH, und die mit dem Alkin umzusetzende Verbindung, vorzugsweise in einer Menge von 0,1 bis 50, besonders bevorzugt 1 bis 30, insbesondere 1 bis 25 mol-% eingesetzt.

Die Temperatur bei der Umsetzung beträgt vorzugsweise -20 bis 200°C, besonders bevorzugt 20 bis 160°C, insbesondere 80 bis 120°C.

Der Druck im Reaktionssystem beträgt vorzugsweise 1 bis 25 bar (abs), besonders bevorzugt 1 bis 16 bar, insbesondere 1 bis 14 bar. Vorzugsweise wird dabei das Alkin kontinuierlich nachgepreßt.

Sofern mit einem Lösungsmittel gearbeitet wird, beträgt der Anteil an Lösungsmittel, bezogen auf die mit dem Alkin umzusetzende Verbindung, vorzugsweise 2 bis 99 Gew.-%, besonders bevorzugt 20 bis 80 Gew.-%.

Die Umsetzung kann kontinuierlich oder diskontinuierlich in allen dafür geeigneten Reaktoren durchgeführt werden. Die Reaktionszeit beträgt in der Regel 0,1 bis 48 Stunden, vorzugsweise 0,1 bis 24 Stunden, insbesondere 0,1 bis 12 Stunden.

Die Erfindung wird durch die nachfolgenden Beispiele näher erläutert, wobei mit* angegebene Ausbeuten nicht optimiert sind:

### Beispiele

### CsOH · H₂O-katalysierte Alkinylierung

### Verfahren A: Umsetzung mit Phenylacetylen (1a)

### Z.B. Herstellung von 2-Ethyl-1-phenyl-1-hexin-3-ol (3n):

In einem 25 l fassenden Schlenk-Kolben wurde CsOH · H₂O (68 g, 0,041 mmol) vorgelegt. THF (10 ml) und Phenylacetylen (660 mg, 6,0 mmol) wurden nacheinander mit einer Spritze zugegeben. Das Reaktionsgemisch wurde stark gerührt und langsam mit 2-Ethylbutyraldehyd (410 mg, 4,1 mmol) versetzt. Das Reaktionsgemisch wurde für eine Stunde bei Raumtemperatur gerührt. Anschließend wurde das Reaktionsgemisch in Ether (150 ml) geschüttet und mit Wasser (2 x 75 ml) und Kochsalzlösung (75 ml) gewaschen. Die Etherphase wurde getrocknet (MgSO₄). Nach Verdampfung des Lösungsmittels wurde der Rückstand durch Flashchromatographie (Pentan/Ethylacetat 9:1) gereinigt, wodurch das gewünschte Produkt (3n) als farbloses Öl (650 mg, 81 %) erhalten wurde.

### Verfahren B: Umsetzungen mit anderen Alkinen

### z.B. Herstellung von 3-Ethyl-5-decyn-1-ol (3o):

In einem 25 ml fassenden Schlenk-Kolben wurde CsOH · H₂O (250 mg, 1,23 mmol) vorgelegt. Ein 1:1-Gemisch von THF und DMSO (10 ml) und Hexin (670 mg, 8,2 mmol) wurden nacheinander mit Hilfe einer Spritze zugegeben. Das Reaktionsgemisch wurde stark gerührt und langsam über eine Spritzenpumpe (0,5 ml/h) mit 2-Ethylbutyraldehyd (410 mg, 4,1 mmol), gelöst in einem 1:1-Gemisch von THF und DMSO (2 ml) versetzt. Das Reaktionsgemisch wurde bei Raumtemperatur für drei Stunden gerührt. Nach der Aufarbeitung gemäß Verfahren A wurde das Produkt (3o) als farbloses Öl (670 mg, 90 %) erhalten.

Ergebnisse für zahlreiche unterschiedlicheElectrophile und Nucleophile sind in der nachstehenden Tabelle I angegeben.

Für unterschiedliche aliphatische Ketone wurden für die entsprechende Umsetzung Ausbeuten von 66 bis 96 % erhalten. In vielen Fällen waren die Umsetzungen innerhalb von fünf Minuten bei Umgebungstemperatur abgeschlossen. Für die Addition einiger Alkine wurde ein 1:1-Gemisch aus THF und DMSO als Lösungsmittel in Verbindung mit CsOH · H₂O (30 mol.-%) eingesetzt. Unter diesen Bedingungen addieren die meisten terminalen Alkine glatt an sekundäre oder tertiäre aliphatische Aldehyde oder aliphatische Ketone zur Bildung der entsprechenden Propargylalkohole des Typs 3 (siehe Tabelle I). Um eine kompetitive Aldotreaktion zu vermeiden, wurde der Aldehyd unter Verwendung einer Pumpe kontinuierlich mit einer Rate von 0,5 l/h zugegeben.

Die Addition von Alkinen an Carbonyl-Verbindungen mit diastereotopen Zentren wie 2-methylcyclohexanon oder ähnlichen Aldehyden führt zu 1:1-Gemischen der diastereomeren Propargylalkohole.

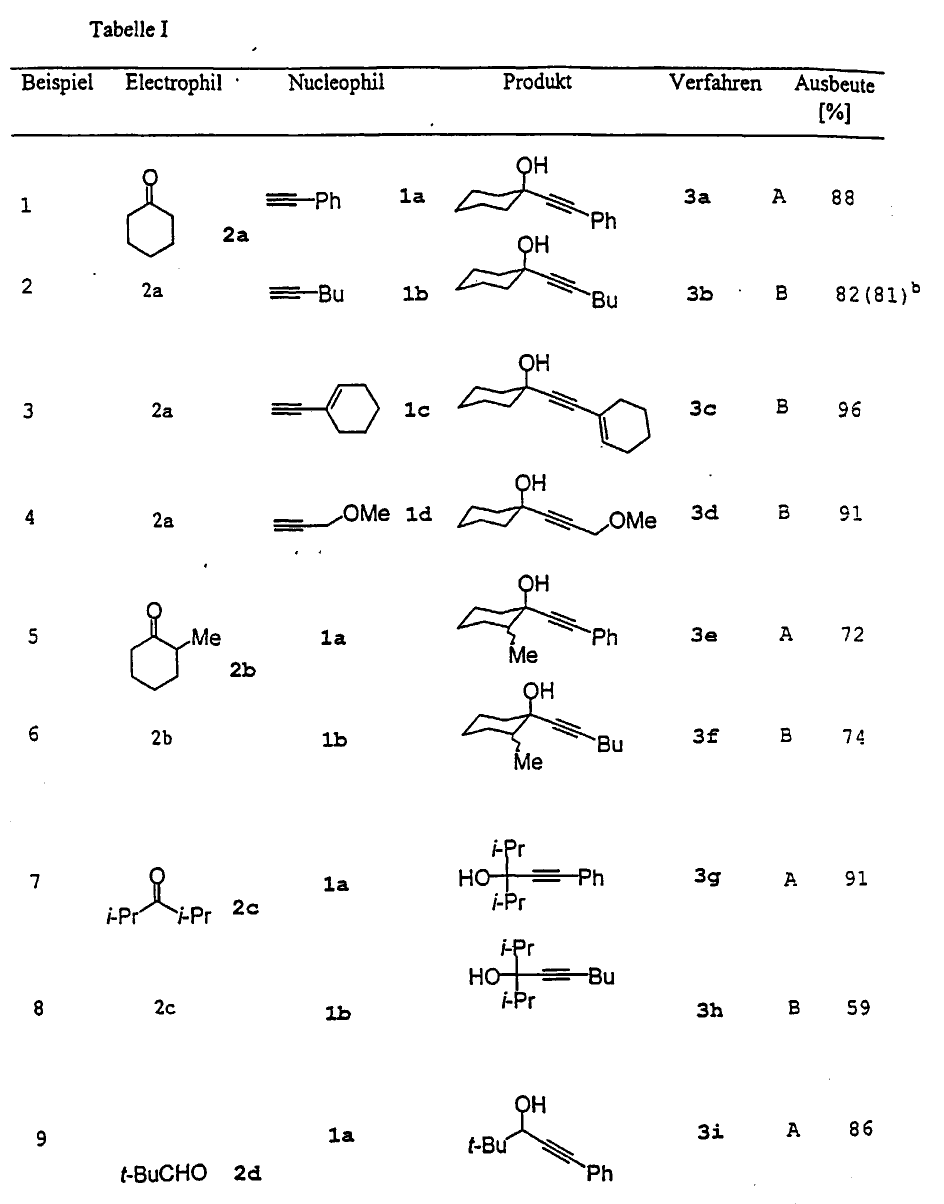

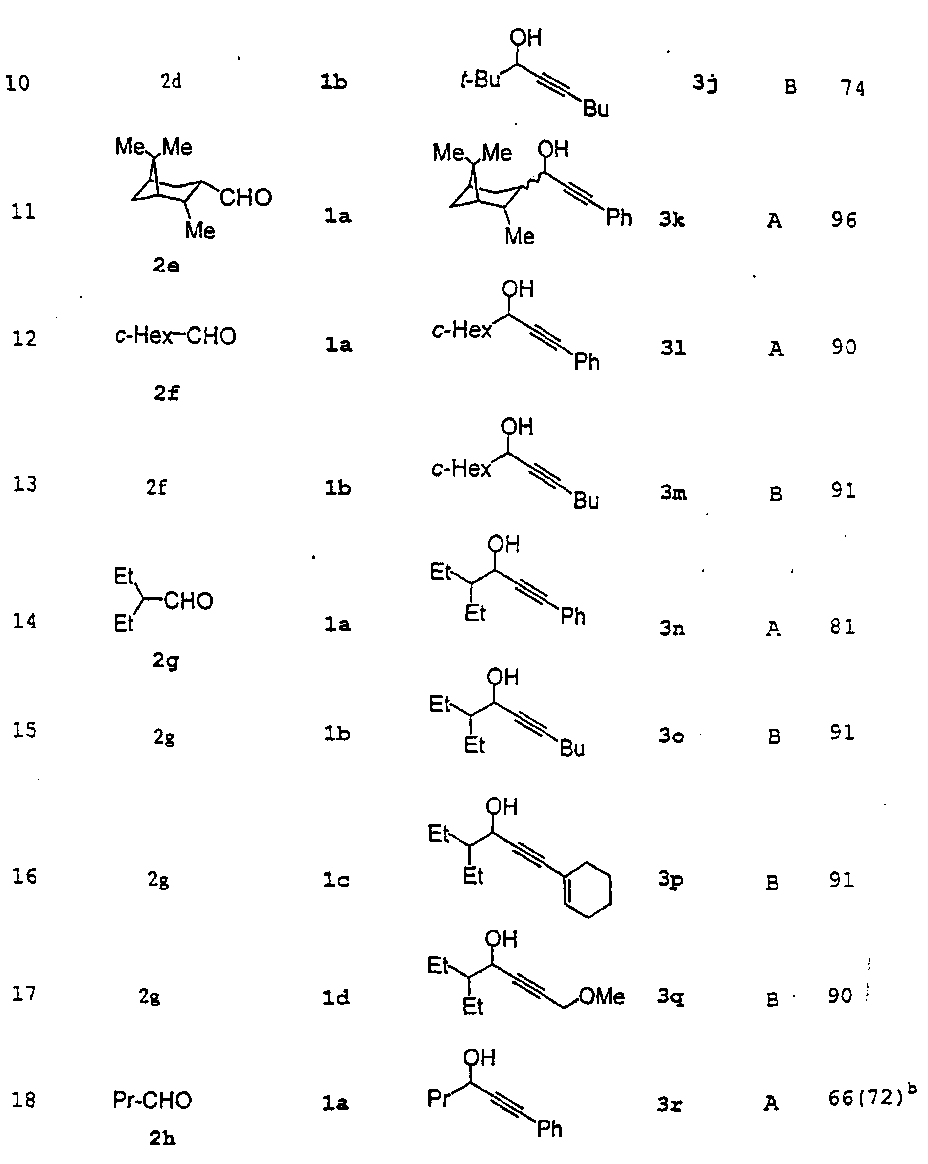

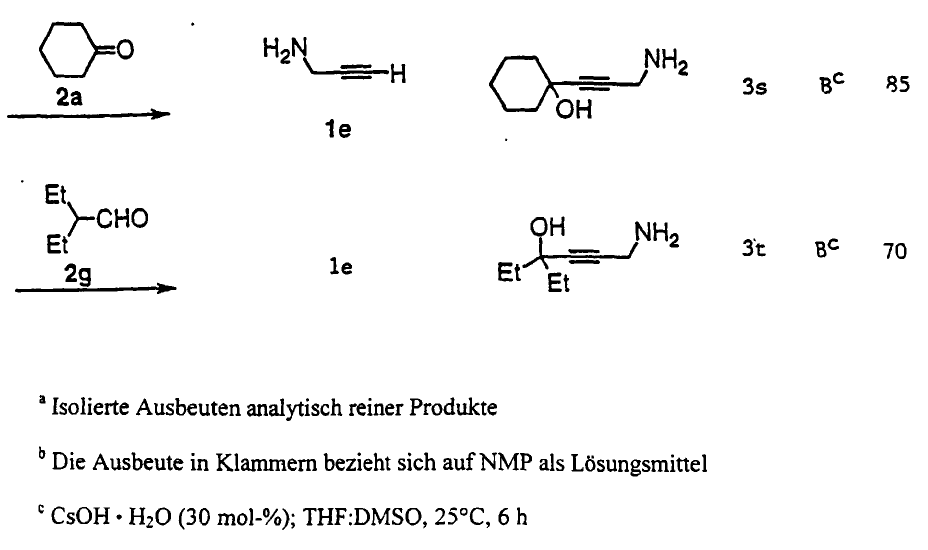

### Beispiel 21

### Alkinylierung von Cyclohexan im System NMP/KOH:

In einer 100 ml Rührapparatur werden 9,8 g Cyclohexan (0,1 Mol) und 1,4 g KOH (0,025 Mol) in 20 ml NMP gelöst. Anschließend wird Acetylen mit einem Volumenstrom von 6 l/h bei Raumtemperatur eingeleitet. Nach 75 min wird die Acetylenzugabe abgestellt und die Reaktionsmischung mit 25 ml H₂O neutralisiert. Die organische Phase wird mit MTBE extrahiert, der Ether schließlich eingdampft. Der Rohaustrag enthält 45 % vom Ethinylierungsprodukt Cyclohexylethinylcarbinol.

### Beispiel 22

### Alkinylierung von 2-Ethylhexanal im System NMP/KOH:

In einer 100 ml Rührapparatur werden 2,1 g KOH (30 Mol-%) in 50 ml NMP gegeben. Anschließend wird Acetylen 1 h lang bei Raumtemperatur eingeleitet. 16 g Ethylhexanal (0,125 Mol) werden innerhalb 1 h bei gleichzeitigem Einleiten von Acetylen zugetropft. Nach einer Nachreaktionszeit von 1 h wird das Reaktionsgemisch mit 50 ml MTBE und 50 ml H₂O versetzt. Die organische Phase wird getrennt, die wäßrige mit MTBE extrahiert, die vereinten organischen Phasen werden eingeengt. Man erhält Ethyloctinol mit einer Ausbeute von 91 %.

### Beispiel 23

### Ethinylierung von Ethylhexanal zu Ethyloctinol im System NMP/KOH

In einer 100 ml Rührapparatur werden 50 ml N-Methylpyrrolidon und 0,7 g KOH (12,3 mmol) vorgelegt. Die Suspension wird bei Raumtemperatur innerhalb 0,5 h mit Acetylen gesättigt und auf 0°C abgekühlt. Bei dieser Temperatur werden unter ständigem Einleiten von Acetylen (15 l/h) 16 g Ethylhexanal innerhalb 1 h zugetropft. Die Nachreaktionszeit bei Raumtemperatur beträgt 1 h. Der Rohaustrag wird mit gleichen Volumenanteilen (mindestens 100 ml) Methyt-tert.-butylether und Wasser versetzt und mit verdünnter Schwefelsäure neutralisiert. Die Phasen werden getrennt, die wäßrige Phase wird dreimal mit 50 ml Methyl-tert.-butylether extrahiert. Die vereinigten organischen Phasen werden eingeengt. Das Lösungsmittel NMP und das Produkt Ethyloctinol werden in einer fraktionierten Destillation gewonnen. Die Ausbeute an Ethyloctinol beträgt 90,1 %.

## Patentansprüche

1. Verwendung von CsOH · H₂O als Katalysator bei der Alkinylierung von organischen Verbindungen, die Gruppen >C=O enthalten, durch Umsetzung der Gruppen mit organischen Verbindungen, die die Gruppe -C≡C-H enthalten.

2. Verfahren zur Herstellung von Alkin-Verbindungen der allgemeinen Formel (I)
R¹R²C(OH)-C≡C-R³ (I)
mit der Bedeutung
R¹, R², R³ unabhängig H oder C₁₋₂₀-Kohlenwasserstoffrest, der ein- oder mehrfach durch C₁₋₆-Alkyl und/oder Halogen substituiert und/oder von nicht benachbarten Heteroatomen unterbrochen sein und/oder C-C-Doppelbindungen enthalten kann, wobei mindestens einer der Reste R¹ und R² von H verschieden ist,
durch Umsetzung von Verbindungen der allgemeinen Formel (II)
R¹-C(=O)-R² (II)
mit Verbindungen der allgemeinen Formel (III)
H-C≡C-R³ (III)
in Gegenwart eines basischen Katalysators, dadurch gekennzeichnet, daß als basischer Katalysator CsOH · H₂O eingesetzt wird und/oder die Umsetzung in einem Lösungsmittel auf Basis von NMP durchgeführt wird.

3. Verfahren nach Anspruch 2, dadurch gekennzeichnet, daß bei Umsetzung in einem Lösungsmittel auf Basis von NMP als basischer Katalysator ein Alkalihydroxid eingesetzt wird.

4. Verfahren nach Anspruch 3, dadurch gekennzeichnet, daß als basischer Katalysator CsOH · H₂O eingesetzt wird.
